# EUROPÄISCHE PATENTSCHRIFT

(11) **EP 1 875 886 B1**
(45) Veröffentlichungstag und Bekanntmachung des Hinweises auf die Patenterteilung: **08.07.2009**
(21) Anmeldenummer: 07108323.2
(22) Anmeldetag: 16.05.2007
(51) Int. Cl.: A61F 7/02, G08B 21/06

(54) **Anti-Schlafgerät**
Anti-sleep device
Appareil anti-sommeil

(30) Priorität: 06.07.2006 DE 202006010486 U
(43) Veröffentlichungstag der Anmeldung: 09.01.2008
(73) Patentinhaber: Pogorelik, Solomon, 47051 Duisburg (DE)
(72) Erfinder: Pogorelik, Solomon, 47051 Duisburg (DE)
(74) Vertreter: Farago, Peter Andreas

(56) Entgegenhaltungen:
- EP-A- 0 929 055
- EP-A2- 0 330 472
- US-A1- 2002 175 821
- US-B1- 6 882 881

## Beschreibung

Die vorliegende Erfindung betrifft allgemein ein Anti-Schlafgerät und insbesondere ein Anti-Schlafgerät mit mindestens einem steuerbaren Peltier-Element.

### Stand der Technik

Bekanntlich ist Müdigkeit bei zahlreichen eintönigen Tätigkeiten, wie z. B. Autofahren, Bahnfahren, Fliegen und dergleichen über längere Strecken ein weit verbreitetes Problem. Als Folge der Müdigkeit kann der so genannte Sekundenschlaf eintreten, der zu einer Gefährdung des Fahrers und allgemein der übrigen Verkehrsteilnehmer führen kann.

Zur Vermeidung des Sekundenschlafs sind aus dem Stand der Technik verschiedene Anti-Schlafgeräte bekannt, die diesen beispielsweise anhand der Stellung des Kopfes oder der Augenlider erkennen und einen entsprechenden akustischen Alarm auslösen.

Bei diesen bekannten Anti-Schlafgeräten hat sich jedoch die Erkennung oder Erfassung der Alarmbedingung als problematisch erwiesen.

US 2002/0175821 A1 offenbart ein Anti-Schlafgerät mit zwei Peltierelementen.

### Zusammenfassung der Erfindung

Angesichts des obigen Problems besteht eine Aufgabe der vorliegenden Erfindung in der Bereitstellung eines Anti-Schlafgeräts, das den Sekundenschlaf wirksam unterbindet.

Diese und weitere der nachstehenden Beschreibung und Zeichnungen zu entnehmenden Aufgaben werden von einem Anti-Schlafgerät gemäß Anspruch 1 gelöst. Weitere vorteilhafte Merkmale der vorliegenden Erfindung sind den dem Anspruch 1 anschließenden Unteransprüchen zu entnehmen.

Dabei basiert das erfindungsgemässe Anti-Schlafgerät auf der physiologischen Wirkung des lymbischen Systems, das bekanntlich den Wach- und Schlafzustand beim Menschen steuert. Gleichzeitig nutzt die vorliegende Erfindung das bekannte Phänomen aus, dass eine Änderung der Aussentemperatur um etwa 8 bis 10 Grad Celsius vom Menschen wahrgenommen wird.

Die Merkmale und Vorteile der vorliegenden Erfindung sowie der Aufbau und die Wirkungsweise der derzeitig bevorzugten Ausführungsform der vorliegenden Erfindung werden unten mit Bezug auf die begleitenden Zeichnungen beschrieben. Die begleitenden Zeichnungen veranschaulichen die vorliegende Erfindung und dienen zusammen mit der Beschreibung weiterhin dazu, die Grundsätze der Erfindung zu erklären und es einem Fachmann auf dem betreffenden Gebiet zu ermöglichen, die Erfindung herzustellen und zu verwenden.

### Kurzbeschreibung der Zeichnungen

Fig. 1 zeigt eine allgemeine schematische Ansicht des Anti-Schlafgeräts gemäß der Erfindung;
Fig. 2 zeigt eine Draufsicht des Kühlkörpers des Anti-Schlafgeräts der Fig. 1; und
Fig. 3 zeigt einen Schnitt entlang der Linie A-A der Fig. 3.

### Beschreibung der bevorzugten Ausführungsform der Erfindung

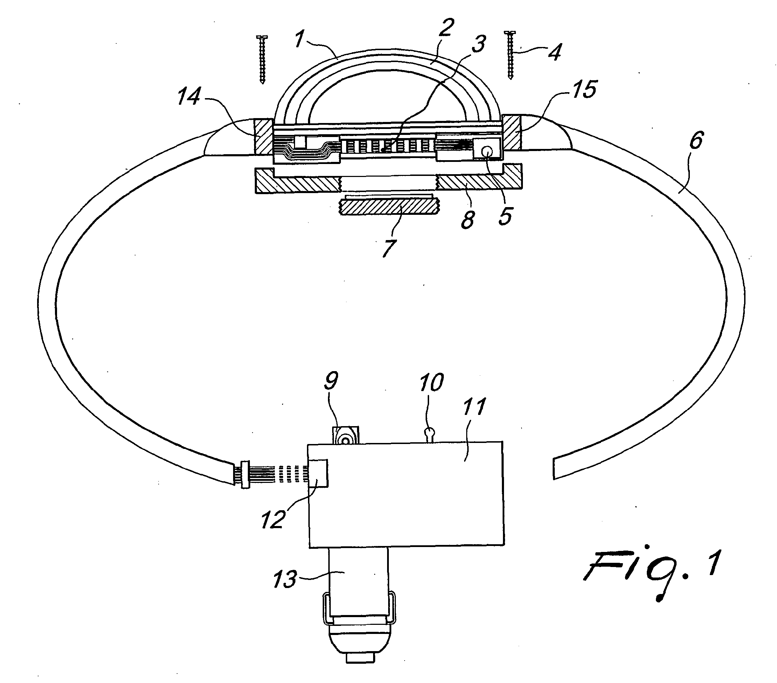

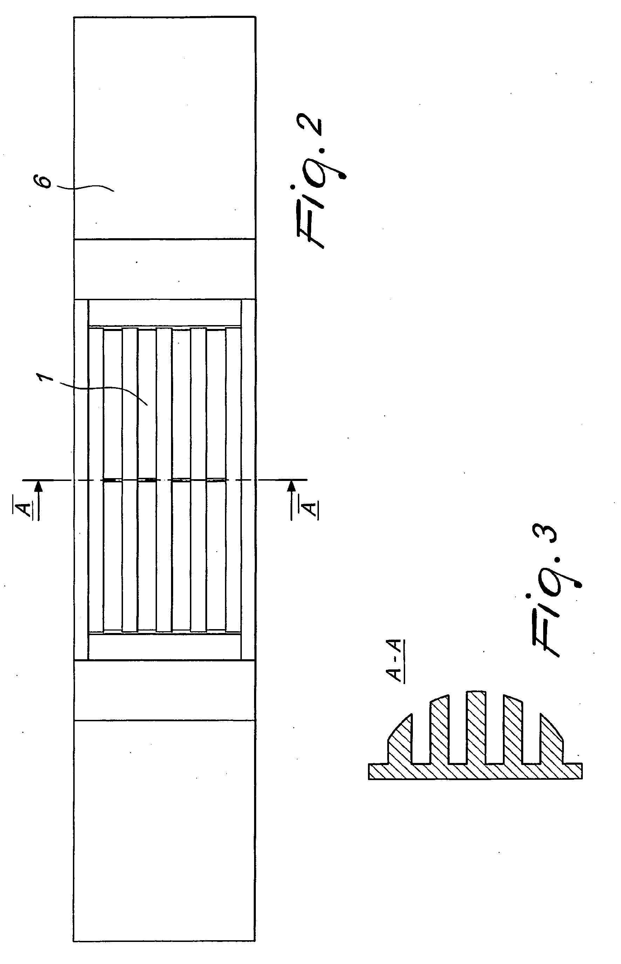

Unter Bezugnahme auf die Figuren 1 bis 3 umfasst das Anti-Schlafgerät der Erfindung einen mit Bezugszeichen 1 bezeichneten Kühlkörper mit einem sich davon seitlich erstreckenden Abschnitt 15, an dem ein Deckel 8 mit Schrauben 15 befestigbar ist. Ein temperaturhemmendes Material 14, das sich zwischen Deckel 8 und Kühlkörper 1 befindet, wird durch den Deckel 8 angepresst und sorgt für minimale Temperaturverluste zwischen Kühlkörper 1 und Deckel 8.

Der Kühlkörper 1 ist an beiden Enden mit einem schematisch dargestellten Stirnband 6 ausgestattet, das durch Klettverschlüsse (nicht gezeigt) an den Enden fixierbar ist, um das Anti-Schlafgerät am Kopf seines Trägers zu fixieren.

Innerhalb des Deckels 8, auf der zum Deckel 8 gewandten Seite des Kühlkörpers 1, ist mindestens ein Peltier-Element 3 vorgesehen, das die erforderliche Temperatur bzw. die erforderlichen Temperaturschwankungen für die Erregung des lymbischen Systems erzeugt und diese über einen am Deckel 8 angebrachten Metallkörper oder Knopf 7 an das lymbische System weitergibt. Die maximale bzw. minimale Höhe der Temperatur, die zur Vermeidung von Verletzungen am Menschen zu wählen ist, wird durch einen Sensor 2 begrenzt. Das Peltier-Element 3 und der Sensor 2 werden am Boden des Kühlkörpers 1 befestigt.

In einer Box 11 befindet sich die Steuerelektronik des Anti-Schlafgeräts das für das thermoelektrische sowie für das mechanisch akustische Geschehen, wie nachstehend erläutert, verantwortlich ist.
Erfindungsgemäß erzeugt die Steuerelektronik mittels eines darin enthaltenen Zufallgenerators elektrische Signale, die das Peltier-Element 3 kontinuierlich anregen. Durch das zufällige Erzeugen der Signale, wird das Peltier-Element 3 kontinuierlich relativ aufgeheizt oder gekühlt, so dass das lymbische System entsprechend angeregt wird und ein Einschlafen des Trägers des Geräts vermieden wird. Der Sensor 2 erfasst die Temperatur am lymbischen System und begrenzt, wie erläutert, die Temperaturschwankungen um Verletzungen des Trägers des Anti-Schlafgeräts bzw. ein Unwohlsein durch übermäßige Schwankungen zu vermeiden. Besonders vorteilhaft wird das Peltier-Element 3 derart angesteuert, dass am lymbischen System Temperaturschwankungen im Bereich von etwa 30 bis 40 Grad Celsius und noch mehr bevorzugt von etwa 31 bis 39 Grad Celsius wahrgenommen werden, wobei wie bei Peltier-Elementen bekannt, die Erhöhung der Temperatur schneller als die Senkung derselben erfolgt. In der Praxis kann eine Erhöhung der Temperatur des Peltier-Elements von etwa 30 bis etwa 40 Grad Celsius in etwa 3 Minuten erfolgen und die Abkühlung von etwa 40 auf 30 Grad Celsius in etwa 8 Minuten stattfinden.

In einer derzeit bevorzugten Ausführungsform der Erfindung erfolgt die Ansteuerung des Peltier-Elements 3 durch eine stufenförmige Spannung mit zwei Pegeln, wobei der untere Pegel einer Spannung entspricht, die das Peltier-Element 3 auf die niedrigere Temperatur (etwa 30 bis 31 Grad Celsius) ansteuert, und der höhere Pegel der Spannungsstufe steuert das Peltier-Element 3 auf die höhere Temepratur von etwa 39 bis 40 Grad Celsius an. Vorzugsweise ist die Verweildauer eines jeden Pegels (bzw. einer jeden Spannungsstufe) abhängig von der vorstehend erläuterten Reaktionszeit des Peltier-Elements auch in einem Bereich von Minuten.

Die Stromversorgung der Steuerelektronik, des Sensors 2 und des Peltier-Elements 3 erfolgt über einen Stecker 12 und einen Adapter 13, der in den Zigarettenanzünder eines Fahrzeugs einführbar ist. Es ist jedoch erfindungsgemäß selbstverständlich denkbar, eine andere Stromquelle zu verwenden wie z. B. eine Batteriezelle, einen Akkumulator oder dergleichen.

In Verwendung wird das Anti-Schlafgerät auf der Stirn des Trägers derart befestigt, dass der temperaturleitende Knopf 7 über dem lymbischen System platziert wird. Danach wird das Anti-Schlafgerät durch die Aktivierung der Stromversorgung in Gang gesetzt, was durch eine Diode 9 angezeigt werden kann. Wie erläutert, arbeitet dann das Peltier-Element 3 kontinuierlich, um zufällige Temperaturschwankungen am lymbischen System in einem Bereich von etwa 30-40 Grad Celsius zu erzeugen, wobei der Sensor 2 dann Schwankungen der Temperatur über diesen Bereich hinaus verhindert.

Neben dem Peltier-Element 3 kann vorteilhaft (in Ergänzung zu diesem) ein akustischer Schlafwarner angebracht werden, der in seinem Inneren eine Kugel 5 enthält, die beim Kippen des Kopfes des Trägers einen elektrischen Kontakt auslöst, wodurch ein akustisches Signal ausgelöst werden kann. Je nach Wunsch des Benutzers/Trägers ist es möglich durch die Änderung der Position des Hebels 10 den akustischen Schlafwarner ab- oder zuzuschalten. Die Stromversorgung des akustischen Schlafwarners erfolgt über den Stecker 12 und Adapter 13 bzw. über eine Batterie oder einen Akkumulator.

Die Erfindung erfüllt vollständig die gestellten Aufgaben, indem ein Anti-Schlafgerät zur Verfügung gestellt wird, das es dem Benutzer/Träger ermöglicht länger wach und konzentriert zu bleiben. Das Anti-Schlafgerät der Erfindung kann besonders vorteilhaft von Fahrern, Piloten, Lokführern, Wachpersonal und dergleichen verwendet werden.

Obwohl die Erfindung in ihren bevorzugten Ausführungsformen beschrieben wurde, sollte es klar sein, dass Änderungen vorgenommen werden können, ohne sich vom Schutzumfang der Erfindung, wie in den anliegenden Ansprüchen definiert, zu lösen.

Wenn Merkmale in den Ansprüchen mit Bezugszeichen versehen sind, so sind diese Bezugszeichen lediglich zum besseren Verständnis der Ansprüche vorhanden. Dementsprechend stellen solche Bezugszeichen keine Einschränkungen des Schutzumfangs solcher Merkmale dar, die nur exemplarisch durch solche Bezugszeichen bezeichnet sind.

## Patentansprüche

1. Anti-Schlafgerät mit einem Kühlkörper (1), mindestens einem am Kühlkörper (1) befestigbaren Peltier-Element (3) und einer Steuerelektronik, die operativ mit dem Peltier-Element (3) verbunden ist, **gekennzeichnet dadurch, dass** die Steuerelektronik einen Zufallsgenerator umfasst, um das Peltier-Element (3) kontinuierlich mit einer Spannung zu beaufschlagen, so dass das lymbische Systems eines Trägers des Anti-Schafgeräts erregt wird.

2. Anti-Schlafgerät nach Anspruch 1, wobei der Zufallsgenerator ausgebildet ist, um die Pegel der Spannung derart zu erzeugen, dass am lymbischen System des Trägers des Anti-Schlafgeräts Temperaturschwankungen in einem Bereich von etwa 30 bis 40 Grad Celsius und vorzugsweise in einem Bereich von etwa 31 bis 39 Grad Celsius wahrgenommen werden.

3. Anti-Schlafgerät nach Anspruch 1 oder 2, wobei der Kühlkörper (1) an beiden Enden mit einem Stirnband (6) ausgestattet ist, um das Anti-Schlafgerät am Kopf seines Trägers zu fixieren.

4. Anti-Schlafgerät nach einem oder mehreren der voranstehenden Ansprüche, das weiterhin einen Sensor (2) umfasst, der ausgebildet ist, um die Temperatur am lymbischen System zu erfassen und diese zu begrenzen.

5. Anti-Schlafgerät nach einem oder mehreren der voranstehenden Ansprüche, das weiterhin einen zuschaltbaren akustischer Schlafwarner umfasst, der in seinem Inneren eine Kugel (5) enthält, die beim Kippen des Kopfes des Trägers einen elektrischen Kontakt auslöst, wodurch ein akustisches Signal erzeugt wird.

6. Anti-Schlafgerät nach einem oder mehreren der voranstehenden Ansprüche, wobei der Kühlkörper (1) mit einem Deckel (8) abschließbar ist, der in seinem Inneren das Peltier-Element (3) enthält, und wobei der Deckel (7) einen Metallkörper (7) umfasst, das die Temperaturschwankungen an das lymbische System weitergibt.

## Claims

1. Anti-sleep device having a cooling body (1), at least one Peltier element (3) which can be fastened to the cooling body (1) and control electronics operatively connected to the Peltier element (3), **characterized in that** the control electronics comprise a random generator for continuously providing the Peltier element (3) with a voltage so that the limbic system of a carrier of the anti-sleep device is excited.

2. Anti-sleep device according to Claim 1, where the random generator is adapted such as to generate the voltage levels in a way such that in the limbic system of the carrier of the anti-sleep device, temperature variations within a range of approximately 30 to 40 degrees Celsius, and preferably within a range of approximately 31 to 39 degrees Celsius, are perceived.

3. Anti-sleep device according to Claim 1 or 2, where the cooling body (1) is provided, on both ends, with a headband (6) for fixing the anti-sleep device to the head of its carrier.

4. Anti-sleep device according to one or more of the above claims, further comprising a sensor (2) which is adapted to detect the temperature in the limbic system and to limit it.

5. Anti-sleep device according to one or more of the above claims, further comprising a connectible acoustic sleep-warning device which has a sphere (5) in its interior which triggers an electrical contact in case of tilting of the carrier's head, resulting in an acoustic signal.

6. Anti-sleep device according to one or more of the above claims, the cooling body (1) being lockable by a lid (8) which contains the Peltier element (3) in its interior, and the lid (7) comprising a metal body (7) which transmits the temperature variations to the limbic system.

## Revendications

1. Appareil anti-sommeil, avec un corps de refroidissement (1), au moins un élément à effet Peltier (3), susceptible d'être fixé sur le corps de refroidissement (1), et un dispositif électronique de commande, relié fonctionnellement à l'élément à effet Peltier (3), **caractérisé en ce que** le dispositif électronique comprend un générateur aléatoire, pour solliciter l'élément à effet Peltier (3) de façon continue avec une tension électrique, de manière que le système lymbique d'un porteur de l'appareil anti-sommeil soit excité.

2. Appareil anti-sommeil selon la revendication 1, le générateur aléatoire étant réalisé pour générer le niveau de la tension, de manière que des fluctuations de température, dans une plage d'à peu près 30 à 40 degrés Celsius et, de préférence, dans une plage d'à peu près 31 à 39 degrés Celsius, soient perçues au système limbique du porteur de l'appareil anti-sommeil.

3. Appareil anti-sommeil selon la revendication 1 ou 2, le corps de refroidissement (1) étant équipé, aux deux extrémités, d'une bande frontale (6), pour fixer l'appareil anti-sommeil sur la tête de son porteur.

4. Appareil anti-sommeil selon l'une ou plusieurs des revendications précédentes, comprenant en outre un capteur (2), réalisé pour appréhender la température au système limbique et limiter celle-ci.

5. Appareil anti-sommeil selon l'une ou plusieurs des revendications précédentes, comprenant en plus un avertisseur d'endormissement acoustique, commutable, contenant intérieurement une bille (5) déclenchant, lors de l'inclinaison de la tête du porteur, un contact électrique, faisant qu'un signal acoustique est produit.

6. Appareil anti-sommeil selon l'une ou plusieurs des revendications précédentes, le corps de refroidissement (1) étant susceptible d'être fermé au moyen d'un couvercle (8), contenant intérieurement l'élément à effet Peltier (3), et où le couvercle (7) comprend un corps métallique (7), retransmettant les fluctuations de température au système limbique.
